Europäisches Patentamt

European Patent Office

Office européen des brevets

(11) Publication number: **0 224 934 B1**

(12) **EUROPEAN PATENT SPECIFICATION**

(45) Date of publication of patent specification: **29.01.92**   (51) Int. Cl.<sup>5</sup>: **A61K 49/00, A61B 8/00**

(21) Application number: **86116943.1**

(22) Date of filing: **05.12.86**

The file contains technical information submitted
after the application was filed and not included in
this specification

(54) **Contrast agent, process for its preparation and its use for ultrasonic imaging.**

(30) Priority: **05.12.85 US 805975**

(43) Date of publication of application:
**10.06.87 Bulletin 87/24**

(45) Publication of the grant of the patent:
**29.01.92 Bulletin 92/05**

(84) Designated Contracting States:
**AT BE CH DE FR GB IT LI NL SE**

(56) References cited:
**EP-A- 0 072 330**
**EP-A- 0 110 502**
**WO-A-80/02365**
**WO-A-84/02838**

**ULTRASONIC IMAGING, vol. 2, 1980, pages
67-77, Academic Press, Inc., US; J. OPHIR et
al.: "Ultrasonic backscatter from contrast
producing collagen microspheres"**

(73) Proprietor: **Feinstein, Steven B.**
**295 Hasting Avenue**
**Higland Park Illinois 60035(US)**

(72) Inventor: **Feinstein, Steven B.**
**295 Hasting Avenue**
**Higland Park Illinois 60035(US)**

(74) Representative: **Patentanwälte Grünecker,
Kinkeldey, Stockmair & Partner
Maximilianstrasse 58
W-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

FIELD OF INVENTION

This invention relates to the field of ultrasonic imaging techniques, and more specifically, to a stabilized ultrasonic imaging agent.

BACKGROUND OF INVENTION

Various technologies exist in which parts of an animal or human body may be imaged so as to aid in diagnosis and therapy. Recently, there have been advances in techniques for ultrasonically imaging various parts of the body; these techniques when applied to the heart in particular are known as "echocardiography." An ultrasonic scanner is used to generate and receive sound waves. The ultrasonic scanner is placed on the body surface overlying the area to be imaged. The sound waves generated by the scanner are directed toward the area to be imaged. The scanner then detects sound waves reflected from the underlying area and translates that data into images.

While such ultrasonic scanners are known in the art, a brief review will be set forth in order to more fully explain the present invention. When ultrasonic energy is transmitted through a substance, the acoustic properties of the substance will depend upon the velocity of the transmissions and the density of the substance. Changes in the substance's acoustic properties (or acoustic impedance) will be most prominent at the interface of different substances (i.e., solids, liquids and gases). As a consequence, when ultrasonic energy is directed through various media, the changes in acoustic properties will change the reflection characteristics, resulting in a more intense sound reflection signal received by the ultra-sonic scanner.

Early ultrasonic imaging techniques such as echocardiograms suffered from a lack of clarity. As a result, extensive efforts were undertaken to improve the ultrasonic scanners and related equipment. In addition, beginning in 1968, "contrast" agents were injected into the bloodstream in an effort to obtain clearer or "enhanced" ultrasonic images. The prior art contrast agents were liquids containing microbubbles of gas, which sometimes were encapsulated with gelatin or saccharine and sometimes were produced by mechanically agitating, i.e., handshaking, mixtures of various liquids. Other prior art contrast agents are disclosed in an article by J. Ophir, et al. entitled "Ultrasonic Backscatter from Contrast Produced by Collagen Micro spheres" in Ultrasonic Imaging by Academic Press, Inc. 1980.

The contrast agents themselves are intense sound wave reflectors because of the acoustic differences between the liquid and the gas microbubbles dissolved therein; thus, when the contrast agents are injected into and perfuse the microvasculature of tissue, clearer images of such tissue may be produced. However, notwithstanding the use of such contrast agents, the image produced, for example of the myocardial tissue, is of relatively poor quality, is highly variable and is not quantifiable due to the variable size and persistence associated with prior art microbubbles. Further, the problems of air emulsion toxicity have not yet been investigated.

In attempting to find a safe, reproducible, quantifiable contrast agent for use in producing an enhanced ultra-sonic image of the tissue under study, researchers have used saccharine and gelatin encapsulated microbubbles of nitrogen or carbon dioxide gas having a mean size of approximately 75 $\mu$m, pressurized gas in liquids (e.g., $H_2O_2$), and mechanically agitated (hand shaken) mixtures of liquid solutions. However, since the pulmonary artery capillaries are about 8 to 10 $\mu$m in diameter, the 75 $\mu$m encapsulated microbubbles may not cross the capillary beds and, as a result, their use would require a direct injection into the area to be imaged or an arterial injection involving the same risks as the invasive approach or angiography discussed above. Further, microbubbles produced by agitating various liquids other than by sonicating them) have wide variability of size. Variable amounts of such non-encapsulated agitated microbubbles can pass through capillaries, but the present state of the art has only produced qualitative data due to the inability to control the variables described above. These contrast agents all work to some degree, but suffer from a number of problems including the fact that the size of the bubbles is not uniform.

My research has demonstrated the feasibility of an improved ultrasonic imaging method as described in published PCT Application WO 84/02838, and in my corresponding United States Patent 4,572,203, issued February 26, 1986. One of my previously disclosed methods utilizes biodegradeable metal-containing microparticles, and another disclosed method utilizes a biocompatible liquid. More specifically, the latter method involves subjecting a biocompatible liquid to high frequency energy in the range of 5000 to 30,000 Hz so as to produce microbubbles having substantially uniform diameter; injecting the bubbles into a mammal to thereby alter the acoustic properties of a predetermined area; and ultrasonically scanning an area including the predetermined area so as to obtain an enhanced image of the predetermined area.

Microbubbles may have a mean particle size of up to 20 $\mu$m. The useable viscous liquid, as disclosed, is to be selected from solutions of dextrose, sorbitol, relatively nontoxic radio-opaque dye, and mixtures thereof.

## SUMMARY OF THE INVENTION

The object of the present invention is to provide an improved ultrasonic imaging agent having smaller and more uniform microbubbles than known imaging agents.

This object is achieved by a stabilized ultrasonic imaging agent obtainable by preparing an aqueous solution of protein or derivatives thereof, subjecting said solution to high frequency sonication to form a dispersion of microbubbles of relatively uniform size therein, the sonication heating said solution and the dispersed bubbles to denature portions of the protein and thereby encapsulate the microbubbles.

A preferred imaging agent comprises albumin as the protein.

In a further preferred embodiment, the stabilized dispersion is formed of microbubbles with a mean bubble diameter of less than 6 $\mu$m, preferably with diameters primarily in the range of 2 to 4 $\mu$m.

In still a further preferred embodiment, the protein is human serum albumin.

In another preferred embodiment, the imaging agent contains the human serum albumin at a concentration of about 5% and the dispersion of microbubbles is formed by sonication which is carried out by applying ultrasonic energy at 5,000 to 30,000 Hz, and preferably at 20,000 Hz

For preparing the stabilized imaging agent of the invention, a viscous solution (e.g., 5% human serum albumin) is subjected to high frequency (5,000 to 30,000 Hz) ultrasonic energy. As a result, microbubbles having a diameter of approximately 2 to 4 $\mu$m are produced. For each of reference such microbubbles will be referred to herein as "sonicated" microbubbles. As described in great detail hereinbelow, such sonicated microbubbles have been found to be improved contrast agents. Alternatively microbubbles comprising protein or derivatives thereof in an aqueous solution are formed into stable contrast agents by any of a number of methods known in the art for physically (via heat) or chemically altering the protein or derivatives to denature or fix the material. For example, the use of heat applied to the contrast agent after formation thereof, or during formation as a result of the sonication of the same is one method for denaturing the protein material to form stable contrast agents. As a second method, fixation (i.e., chemical denaturation) of the protein material using formaldehyde or glutaraldehyde may also be utilized to form stable contrast agents.

Thus, while overcoming many of the problems associated with the prior art, the present invention makes possible the production of unique images of various organ systems. Although the invention technique is applicable to various animal and human body organ systems, its novel features and advantages may be better understood from the following description of its use in obtaining images of myocardial tissue and perfusion or blood flow patterns.

In reviewing the description it should be kept in mind that the heart is a "pump" fed by many blood vessels which, during the course of time, may become partially or totally blocked, causing damage to the heart tissue. In the past, information concerning the heart tissue was obtained using radionuclide imaging or surgery; the angiogram produced no direct data regarding the tissue, but rather required the drawing of inferences from data obtained with respect to the major blood vessels and wall motions of the heart.

## DETAILED DESCRIPTION OF THE INVENTION

The method using the ultrasonic imaging agent of this invention uses equipment like that described in my prior U.S. Patent 4,572,203. This comprises ultrasonic scanning equipment consisting of a scanner and imaging apparatus. The equipment produces visual images of a predetermined area, in this case, the heart region of a human body. Typically, the scanner is placed directly on the skin over the area to be imaged. The scanner houses various electronic components including ultrasonic transducers. The scanner produces ultrasonic waves which perform a sector scan of the heart region. The ultrasonic waves are reflected by the various portions of the heart region and are received by the generating transducer and processed in accordance with pulse-echo methods known in the art. After processing, signals are sent to the imaging apparatus (also well known in the art) for viewing.

In the method using the ultrasonic imaging agent of the present invention, after the patient is "prepped" and the scanner is in place, the sonicated microbubble contrast agent is injected, for example, through an arm vein. The contrast agent flows through the vein to the right venous side of the heart, through the main pulmonary artery leading to the lungs, across the lungs, through the capillaries, into the pulmonary vein and finally into the left atrium and the left ventricular cavity of the heart.

The present invention is directed to both sonicated microbubbles. While not to be bound by any theory,

EP 0 224 934 B1

the sonicated microbubbles of the present invention produce noticeably clearer and more detailed images of the myocardial tissue and microvasculature. This has been demonstrated experimentally. The microbubbles were injected into the pulmonary artery of a dog and have crossed the capillary beds of the lung to enter the left atrium and the left ventricular cavity into the aorta through the coronary arteries and eventually into the left ventricular tissue enhancing the image thereof.

With the method using the ultrasonic imaging agent of this invention, observations and diagnoses can be made with respect to the amount of time required for the blood to pass through the lungs, blood flow patterns, the size of the left atrium, the competence of the mitral valve (which separates the left atrium and left ventricle), chamber dimensions in the left ventricular cavity, and wall motion abnormalities. Upon ejection of the contrast agent from the left ventricle, the competence of the aortic valve also may be analyzed, as well as the ejection fraction or percentage of volume ejected from the left ventricle. Finally, the contrast patterns in the tissue will indicate which areas, if any, are not being adequately perfused.

In summary, such a pattern of images will help diagnose unusual blood flow characteristics within the heart, valvular competence, chamber sizes and wall motion, and will provide a potential indicator of myocardial perfusion.

The stabilized ultrasonic imaging agent of the invention is obtained by preparing an aqueous solution of protein or derivatives thereof, subjecting said solution to high frequency sonication to form a dispersion of microbubbles of relatively uniform size therein, the sonication heating said solution and the dispersed bubbles to denature portions of the protein and thereby encapsulate the microbubbles.

In a presently preferred embodiment of the invention, a solution of protein or derivatives thereof, capable of forming microbubbles when sonicated in accordance with the above-described procedure, is used. One example of a useful solution is a 5% aqueous solution of human serum albumin, referred to herein as albumin. Albumin in solution is commercially available from any of a number of sources. While not being bound by any particular theory of operation, it appears that sonication of the solution under conditions discussed above causes the formation of microbubbles. The resulting microbubbles are substantially different from those described above in that the walls of the microbubbles are significantly more stable, thereby making the microbubbles themselves more stable. The stability of these microbubbles is believed to be a result of the fact that the sonicator heats the albumin to a temperature sufficient to denature the protein. The sonication also creates bubbles primarily in the range of 2-4 $\mu$m. The size distribution of microbubbles formed, as described above, out of a commercially available aqueous solution of 5% albumin was studied. Substantially all of the microbubbles were in the range of 2-4 $\mu$m, as determined by a Coulter Counter, using techniques well-known in the art. Of the microbubbles produced, approximately 8 million per milliliter (ml.) of solution are in the 2-4 $\mu$m range, approximately 1 million microbubbles per ml. in the 4-5 $\mu$m range, less than 0.5 million microbubbles per ml. in the 5-6 $\mu$m range, and relatively negligible amounts of microbubbles in the range above 6 $\mu$m are formed.

As an alternative to heat treatment of the microbubbles as a result of sonication, the protein can be denatured and the microbubbles stabilized by heat treatment to a temperature in the range of 50 to 60° Centigrade, with the actual temperature in the range depending on the protein, proteins used or protein derivatives used. The specific temperature and conditions for denaturation of the various proteins which may be used for the present invention are generally known in the art.

The microbubbles formed from 5% albumin may, in the alternative, be stabilized to form a commercially, clinically usable contrast agent by treatment with various chemical agents which chemically denature, or "fix", the protein, and derivatives thereof. Chemical denaturation of the protein (or derivatives) may be accomplished by either binding the protein with a protein-reactive aldehyde, such as glutaraldehyde. For the latter procedure of stabilizing the invented microbubble contrast agent, the microbubbles may be reacted with 0.25 grams of 50% aqueous glutaraldehyde per gram of protein at pH4.5 for 6 hours. The treated contrast agent is then gently and extensively washed to remove as much of the unreacted glutaraldehyde as possible.

The microbubbles formed from 5% albumin which has been sonicated as described are stabilized and exist for 48 hours or longer. This may be compared with the above-described sonicated sugar solutions which last a few minutes to a few hours. Thereafter, they are no longer effective contrast agents.

This invented echo contrast agent permits left heart imaging from intravenous injections. The sonicated albumin microbubbles, when injected into a peripheral vein are capable of transpulmonary passage. This results in echocardiographic opacification of the left ventricle (LV) cavity as well as myocardial tissue. The sonicated albumin microbubbles are small, stable and echo reflective targets.

A total of 72 intravenous injections of sonicated albumin microbubbles were performed in 5 dogs. Three to 10 ml of contrast solution, containing a minimum of 500,000 bubbles per ml, were injected into the dorsal forepaw vein in each trial. No significant changes were noted in heart rate, blood pressure or arterial blood

4

gases. LV cavity opacification was graded from 0 (no opacification) to +3 (full LV opacification) with the duration noted in seconds. The overall successful transpulmonary opacification rate was 78% (56/72 trials). LV tissue opacification was always preceded by +3 LV capacity opacification. Successful transpulmonary passage of the sonicated albumin microspheres was observed if (a) the RV contrast opacification was +3, (b) the average sphere size was 4 $\mu$ms, or less, and (c) the sphere concentration was at least one million per milliliter. The results are set forth below in Table 1.

## TABLE 1

| LV Cavity Opacification | | Contrast in LV |
|---|---|---|
| Grade | Trials | cavity (seconds) |
| +3 | 11 | 20 $\pm$ 8 |
| +2 | 14 | 18 $\pm$ 8 |
| +1 | 31 | 12 $\pm$ 17 |
| 0 | 16 | 0 |

Thus, as shown here, successful opacification of the LV cavity and myocardial tissue is now feasible using peripheral venous injections of sonicated albumin microspheres.

Besides the scanner briefly described above, there exist other ultrasonic scanners, examples of which are disclosed in U.S. Patent Nos. 4,143,554 and 4,315,435, Basically, these patents relate to various techniques including dynamic cross-sectional echography (DCE) for producing sequential two-dimensional images of cross-sectional slices of animal or human anatomy by means of ultrasound energy at a frame rate sufficient to enable dynamic visualization of moving organs. Types of apparatus utilized in DCE are generally called DCE scanners and transmit and receive short, sonic pulses in the form of narrow beams or lines. The reflected signals' strength is a function of time, which is converted to a position using a nominal sound speed, and is displayed on a cathode ray tube or other suitable devices in a manner somewhat analogous to radar or sonar displays. While DCE can be used to produce images of many organ systems including the liver, gall bladder, pancreas and kidney, it is frequently used for visualization of tissue and major blood vessels of the heart.

The bubbles may be used for imaging a wide variety of areas, even when injected at a peripheral venous site. Those areas include (without limitation): (1) the venous drainage system to the heart; (2) the myocardial tissue and perfusion characteristics during an exercise treadmill test or the like, and (3) myocardial tissue after an oral ingestion or intravenous injection of drugs designed to increase blood flow to the tissue. Additionally, the microbubbles may be useful in delineating changes in the myocardial tissue perfusion due to interventions such as (1) coronary artery vein grafting; (2) coronary artery angioplasty (balloon dilation of a narrowed artery); (3) use of thrombolytic agents (such as streptokinase) to dissolve clots in coronary arteries; or (4) perfusion defects or changes due to a recent heart attack.

Furthermore, at the time of a coronary angiogram (or a digital subtraction angiogram) an injection of the microbubbles may provide data with respect to tissue perfusion characteristics that would augment and complement the data obtained from the angiogram procedure, which identifies only the anatomy of the blood vessels.

Through the use of the microbubbles of the present invention, other non-cardiac organ systems including without limitation the liver, spleen, kidney, etc. that are presently imaged by ultrasonic techniques may be susceptible to an enhancement of such currently obtainable images, and/or or the generation of new images showing perfusion and flow characteristics that had not previously been susceptible to imaging using prior art ultrasonic imaging techniques.

## Claims

**Claims for the following Contracting States:BE, CH, DE, FR, GB, IT, LI, NL, SE**

1. A stabilized ultrasonic imaging agent obtainable by preparing an aqueous solution of protein or derivatives thereof, subjecting said solution to high frequency sonication to form a dispersion of microbubbles of relatively uniform size therein, the sonication heating said solution and the dispersed bubbles to denature portions of the protein and thereby encapsulate the microbubbles.

2. The imaging agent of claim 1 characterized by preparing said aqueous solution from albumin.

3. The imaging agent of claims 1 or 2 characterized by forming said stabilized dispersion of microbubbles with a mean bubble diameter of less than 6 $\mu$m.

4. The imaging agent of any of claims 1 to 3 characterized by forming said stabilized dispersion of microbubbles with diameters primarily in the range of 2 to 4 $\mu$m.

5. The imaging agent of any of claims 1 to 4 characterized in that said protein is human serum albumin.

6. The imaging agent of claim 5 characterized in that said human serum albumin is at a concentration of 5%, said sonication is carried out by applying ultrasonic energy at 5,000 to 30,000 Hz, and preferably at 20,000 Hz.

**Claims for the following Contracting State: AT**

1. A method for preparing a stabilized ultrasonic imaging agent comprising the steps of preparing an aqueous solution of protein or derivatives thereof, subjecting said solution to high frequency sonication to form a dispersion of microbubbles of relatively uniform size therein, the sonication heating said solution and the dispersed bubbles to denature portions of the protein and thereby encapsulate the microbubbles.

2. A process according to Claim 1 wherein said aqueous solution is prepared from albumin.

3. The process according to Claim 1 or 2 wherein said stabilized dispersion is formed of microbubbles with a mean bubble diameter of less than 6 um.

4. The process according to any of Claims 1 to 3 wherein said stabilized dispersion is formed of microbubbles with diameters primarily in the range of 2 to 4 um.

5. The process according to any of Claims 1 to 4 wherein the protein is human serum albumin.

6. The process according to Claim 5 wherein the human serum albumin is at a concentration of 5% and said sonication is carried out by applying ultrasonic energy at 5,000 to 30,000 Hz, and preferably at 20,000 Hz.

**Revendications**

**Revendications pour les Etats contractants suivants: BE, CH, DE, FR, GB, IT, LI, NL, SE**

1. Agent stabilisé de génération d'images par balayage par ultrasons, pouvant s'obtenir en préparant une solution aqueuse de protéine ou de ses dérivés, en soumettant ladite solution à un traitement par les ultrasons à haute fréquence pour y former une dispersion de microbulles de dimension relativement uniforme, le traitement par ultrasons chauffant ladite solution et les bulles dispersées pour dénaturer les portions de la protéine et de ce fait encapsuler les microbulles.

2. Agent de génération d'images de la revendication 1, caractérisé par le fait que l'on prépare ladite solution aqueuse à partir d'albumine.

3. Agent de génération d'images des revendications 1 ou 2, caractérisé par le fait que l'on forme ladite dispersion stabilisée de microbulles avec un diamètre moyen des bulles inférieur à 6 $\mu$m.

4. Agent de génération d'images de l'une quelconque des revendications 1 à 3, caractérisé par le fait que l'on forme ladite dispersion stabilisée de microbulles avec des diamètres principalement situés dans la plage de 2 à 4 $\mu$m.

5. Agent de génération d'images de l'une quelconque des revendications 1 à 4, caractérisé par le fait que ladite protéine est de l'albumine de sérum humain.

6. Agent de génération d'images de la revendication 5, caractérisé par le fait que ladite albumine de sérum humain se trouve à une concentration de 5%, et que ledit traitement par les ultrasons s'effectue en appliquant de l'énergie ultrasonique à une fréquence de 5000 à 30000 Hz et de préférence à 20000 Hz.

**Revendications pour l'Etat contractant suivant: AT**

1. Procédé de préparation d'un agent stabilisé de génération d'images par balayage aux ultrasons, comportant les étapes consistant à préparer une solution aqueuse de protéine ou de ses dérivés, à soumettre ladite solution à un traitement par les ultrasons à haute fréquence pour y former une dispersion de microbulles de dimension relativement uniforme, le traitement par ultrasons chauffant ladite solution et les bulles dispersées pour dénaturer les portions de la protéine et de ce fait encapsuler les microbulles.

2. Procédé selon la revendication 1, dans lequel ladite solution aqueuse est préparée à partir de l'albumine.

3. Procédé selon la revendication 1 ou 2, dans lequel ladite dispersion stabilisée est formée de microbulles d'un diamètre moyen des bulles inférieur à 6 $\mu$m.

4. Procédé selon l'une quelconque des revendications 1 à 3, dans lequel ladite dispersion stabilisée est formée de microbulles avec des diamètres principalement situés dans la plage de 2 à 4 $\mu$m.

5. Procédé selon l'une quelconque des revendications 1 à 4, dans lequel la protéine est de l'albumine de sérum humain.

6. Procédé selon la revendication 5, dans lequel l'albumine de sérum humain se trouve à une concentration de 5%, et ledit traitement par les ultrasons s'effectue en appliquant de l'énergie ultrasonique à une fréquence de 5000 à 30000 Hz et de préférence à 20 000 Hz.

**Patentansprüche**
**Patentansprüche für folgende Vertragsstaaten: BE, CH, DE, FR, GB, IT, LI, NL, SE**

1. Ein stabilisiertes Kontrastmittel für die Ultraschalldarstellung, erhältlich durch Herstellen einer wässrigen Lösung eines Proteins oder eines Derivats davon, Behandeln der Lösung mit Ultraschall, um eine Disperson aus Mikrobläschen relativ gleichförmiger Größe darin zu bilden, wobei die Beschallung die Lösung und die dispergierten Bläschen erwärmt, um Teile des Proteins zu denaturieren und dadurch die Mikrobläschen einzuschließen.

2. Das Kontrastmittel nach Anspruch 1, **gekennzeichnet durch** Herstellen der wässrigen Lösung aus Albumin.

3. Das Kontrastmittel nach Anspruch 1 oder 2, **gekennzeichnet durch** Bilden der stabilisierten Dispersion aus Mikrobläschen mit einem mittleren Bläschendurchmesser von weniger als 6 $\mu$m.

4. Das Kontrastmittel nach einem der Ansprüche 1 bis 3, **gekennzeichnet durch** Bilden der stabilisierten Dispersion aus Mikrobläschen mit Durchmessern in dem Bereich von primär 2 - 4 $\mu$m.

5. Das Kontrastmittel nach einem der Ansprüche 1 bis 4, **dadurch gekennzeichnet**, daß das Protein humanes Serumalbumin ist.

6. Das Kontrastmittel nach Anspruch 5, **dadurch gekennzeichnet**, daß das humane Serumalbumin in einer Konzentration von 5 % vorliegt, wobei die Beschallung ausgeführt wird durch Anwenden von Ultraschallenergie von 5000 - 30000 Hz, und vorzugsweise von 20000 Hz.

**Patentansprüche für folgenden Vertragsstaat: AT**

7

1.  Ein Verfahren zum Herstellen eines stabilisierten Kontrastmittels für die Ultraschalldarstellung, umfassend die Schritte: Herstellen einer wässrigen Lösung eines Proteins oder eines Derivats davon, Behandeln der Lösung mit Ultraschall, um eine Dispersion aus Mikrobläschen relativ gleichförmiger Größe darin zu bilden, wobei die Beschallung die Lösung und die dispergierten Bläschen erwärmt, um Teile des Proteins zu de denaturieren und dadurch die Mikrobläschen einzuschließen.

2.  Ein Verfahren nach Anspruch 1, worin die wässrige Lösung aus Albumin hergestellt wird.

3.  Das Verfahren nach Anspruch 1 oder 2, worin die stabilisierte Dispersion aus Mikrobläschen mit einem mittleren Bläschendurchmesser von weniger als 6 $\mu$m gebildet wird.

4.  Das Verfahren nach einem der Ansprüche 1 bis 3, worin die stabilisierte Dispersion aus Mikrobläschen mit Durchmessern in dem Bereich von primär 2 bis 4 $\mu$m gebildet wird.

5.  Das Verfahren nach einem der Ansprüche 1 bis 4, worin das Protein humanes Serumalbumin ist.

6.  Das Verfahren nach Anspruch 5, worin das humane Serumalbumin in einer Konzentration von 5 % vorliegt und wobei die Beschallung ausgeführt wird durch Anwenden von Ultraschallenergie von 5000 bis 30000 Hz, und vorzugsweise von 20000 Hz.